# EUROPÄISCHE PATENTSCHRIFT

(11) **EP 3 075 348 B1**
(45) Veröffentlichungstag und Bekanntmachung des Hinweises auf die Patenterteilung: **08.05.2019**
(21) Anmeldenummer: 16000548.4
(22) Anmeldetag: 07.03.2016
(51) Int. Cl.: A61D 17/00, A01K 29/00

(54) **VERFAHREN UND EINRICHTUNG ZUM ANZEIGEN EINER ALS WAHRSCHEINLICH LAHMEND AUS EINEM BESTAND ZU SELEKTIERENDEN MILCHKUH**
METHOD AND DEVICE FOR DISPLAYING A DAIRY COW WHICH IS PROBABLY LIMPING AND SELECTED FROM A GROUP
PROCEDE ET DISPOSITIF D'AFFICHAGE D'UNE VACHE LAITIERE VRAISEMBLABLEMENT BOITEUSE A SELECTIONNER A PARTIR D'UN TROUPEAU

(30) Priorität: 04.04.2015 DE 102015004451
(43) Veröffentlichungstag der Anmeldung: 05.10.2016
(73) Patentinhaber: Solnovis GmbH, 91330 Eggolsheim (DE)
(72) Erfinder: Schmitt, Katharina, 90616 Neuhof a.d. Zenn (DE); Winkler, Martin, 91083 Baiersdorf-Igelsdorf (DE)
(74) Vertreter: Führing, Dieter

(56) Entgegenhaltungen:
- WO-A1-2013/052001
- POURSABERI A ET AL: "Real-time automatic lameness detection based on back posture extraction in dairy cattle: Shape analysis of cow with image processing techniques", COMPUTERS AND ELECTRONICS IN AGRICULTURE, ELSEVIER, AMSTERDAM, NL, Bd. 74, Nr. 1, 1. Oktober 2010 (2010-10-01), Seiten 110-119, XP027317583, ISSN: 0168-1699 [gefunden am 2010-09-22]
- AHMAD POURSABERI ET AL: "Online lameness detection in dairy cattle using Body Movement Pattern (BMP)", INTELLIGENT SYSTEMS DESIGN AND APPLICATIONS (ISDA), 2011 11TH INTERNATIONAL CONFERENCE ON, IEEE, 22. November 2011 (2011-11-22), Seiten 732-736, XP032086142, DOI: 10.1109/ISDA.2011.6121743 ISBN: 978-1-4577-1676-8

## Beschreibung

Die Erfindung betrifft ein Verfahren und eine Einrichtung gemäß dem jeweiligen Oberbegriff der unabhängigen Patentansprüche.

Es ist in der milchproduzierenden Landwirtschaft bekannt, dass schmerzhafte Klauenerkrankungen bei Milchkühen, deren Folge das Tier durch eine unnatürliche Verlagerung der Gewichtsbelastung der Beine zu entgehen sucht, zu Lähmungserscheinungen führen. Aus denen resultiert nicht nur ein gestörter Gang der Kuh, sondern insbesondere auch eine zunehmend konvexen Verkrümmung ihrer abhängig von der Rasse originär leicht ansteigenden oder abfallenden, jedenfalls etwa ebenen Rückenkontur in Seitenansicht. Derartige Strapazen mindern die Milchleistung hochleistungsfähiger Milchkühe und sind oft Ursachen von Folgeerkrankungen wie insbesondere Euterentzündungen. Die müssen sehr kosten- und zeitaufwändig behandelt werden, um weitere Leistungseinbußen bis zum Milchausfall möglichst zu vermeiden.

Als Abhilfe ist nach der EP 1 284 649 B1 vorgesehen, in einem schmalen Gang auf dem Weg zur Futterstelle am Boden vier Kraftsensoren zu installieren und aus einer statistischen Auswertung ungleicher Gewichtsverteilung bei der Passage auf eine Erkrankung der Kuh zu schließen. Für belastbare statistische Auswertung müssen aber sehr viele Passagen der selben Kuh erfasst werden, was erst im Verlaufe einer längeren Zeitspanne erreicht ist; und die Korrelation der Messwerte bleibt doch sehr gering angesichts des unsteten Bewegungsverhaltens einer Kuh.

Deshalb ist nach der WO 2013/052001 A1 vorgesehen, eine Box mit einer nach Eintritt einer Kuh hinter deren Hinterbeine verschwenkbaren Photokamera auszustatten, um den unteren Bereich der Beine dieser Kuh zu erfassen. Wenn eines der Beine etwas angehoben ist, so dass diese Klaue nicht vollflächig eben auf dem Untergrund steht, soll das eine Warnung vor einer möglicherweise sich entwickelnden Lähmung sein. Für reproduzierbare, untereinander vergleichbare Aufnahmen muss die Kuh allerdings auf einem harten, ebenen, gekehrten Boden stehen. Und die Vergleichbarkeit aufeinanderfolgender Aufnahmen mit derselben Kuh ist schon in Frage gestellt, wenn die Kuh sich bewegt, etwa nur um das Standbein zu wechseln.

Der Einfluss von klauenerkrankungsbedingten Lähmungserscheinungen steht bei den Leistungseinbußen von Hochleistungskühen an dritter Stelle (nach Euterentzündungen und Fruchtbarkeitsdefizit). Deshalb ist der Milchwirt bemüht, aus der Beobachtung des Bewegungsverhaltens und des Einsetzens der Rückenverkrümmung einer Kuh im Vergleich zu anderen Tieren derselben Rasse möglichst früh, jedenfalls rechtzeitig vor Leistungseinbußen auf Lähmungserscheinungen und daraus resultierend auf das eventuelle Erfordernis einer Klauenbehandlung zu schließen. Die zielgerichtete Beobachtung größerer Bestände ist allerdings aufwändig, zumal mit der Verbreitung vollautomatischer Melkanlagen der frühere direkte Kontakt zwischen einem Melker und seinen Kühen verloren gegangen ist. Andererseits ist der Personal- und Schulungsbedarf vergleichsweise sehr hoch, der für ein anzustreben ununterbrochenes, jedenfalls möglichst häufiges Beobachten und aussagekräftiges Bewerten des Bewegungsverhaltens der Kühe im Laufstall mit wechselndem Personaleinsatz anfällt.

Dagegen wird gemäß den Beiträgen A. Poursaberi et al "Real-time automatic lameness detection based on back posture extraction in dairy cattle: Shape analysis of cow with image processing techniques", COMPUTER AND ELECTRONICS IN AGRICULTURE: ELSEVIER, AMSTERSAM, NL, Bd. 74, Nur.1, 01. October 2010, pp 110-119, ISSN: 0168-1699 beziehungsweise A. Poursaberi et al "Online Lameness detection in dairy cattle using Body Movement Pattern (BMP)" INTELLIGENT SYSTEM DESIGN AND APPLICATIONS (ISDA) 2011, 11th International Conference on, IEEE, 22. November 2011, pp 732-736, ISBN: 978-1-4577-1676-8 vorgesehen, die Kühe auf ihrem Weg vom Stall zur Weide durch einen eigens dafür erstellten schmalen Vereinzelungs-Korridor zu schicken und dabei seitlich zu filmen. Aus diesem Bewegungs-Filmmaterial werden dann jeweils mehrere Standbilder derselben Kuh vereinzelt und so ausgeblendet, dass sie sich nur noch über deren Rücken, beziehungsweise auch noch über den Nacken bis zum Kopf hin, erstrecken. Diese Bilder werden, nach einer Grauwertumsetzung, mit einer Anfangs-Referenzaufnahme noch ohne Kuh verglichen, um dann aus mehreren Binärbildern unter Einsatz von Filteralgorithmen eine repräsentative Rückenlinie zu extrahieren; wozu gegebenenfalls eine mehrfache Ellipsenüberlagerung konstruiert wird. Die Ergebnisse würden allerdings variieren je nach wechselnden Schattenwürfen auf und hinter den gefleckten Kühen; auch sei das Bewegungsverhalten der Kühe und dadurch deren Körperhaltung im Korridor durch nachdrängende Tiere stark beeinflusst.

Angesichts solcher Gegebenheiten beruht vorliegende Erfindung auf der Erkenntnis, dass die gelegentliche Beobachtung des Bewegungsverhaltens einzelner Kühe für eine statistische Schlussfolgerung nicht mehr repräsentativ genug ist; zumal unter Bewegung der Kuh Einflüsse, die nicht krankheitsbedingt sein müssen, sich auf das Erscheinungsbild der Kuh auswirken können.

Deshalb liegt vorliegender Erfindung die technische Problemstellung zugrunde, mittels einer selbständig arbeitenden Einrichtung Lähmungserscheinungen aufweisende Milchkühe eines Bestandes anzuzeigen; um diese Kühe dann gezielt selektieren und einer Untersuchung hinsichtlich des Klauenzustandes und davon abhängiger Bewegungsbeeinträchtigungen zuführen zu können, ehe ihre Milchleistung nachlässt.

Diese Aufgabe ist gemäß den in den unabhängigen Patentansprüchen angegebenen wesentlichen Merkmalen gelöst. Danach wird aus einer photographierten Seitenansicht der jeweiligen Kuh mittels einer handelsüblichen Bildverarbeitungs-Einrichtung der Verlauf und damit auch die Krümmung der Rückenlinie der in wiederkehrender, annähernd definierter Stellung möglichst ruhig stehenden Milchkuh vermessen. Eine gegebenenfalls vorliegende Krümmung dieses Verlaufes wird von der Einrichtung als mögliche oder gar wahrscheinliche Lähmung der mittels Ohrmarke oder z.B. Transponder-Bestückung individualisierten Kuh im Vergleich zu vorangegangenen Messungen an dieser Kuh protokolliert. Wenn auf diese Weise eine Lahmheit festgestellt und in Zuordnung zur jeweiligen Kuh angezeigt ist, kann wenigstens zeitnah eine nähere Diagnose erfolgen und eine Behandlung einsetzen.

Um mit möglichst wenig apparativem und datenverarbeitungstechnischem Aufwand vergleichbare Ergebnisse zu erzielen, werden die Kühe jeweils bei Stillstand in der selben Umgebung von einer starr montierten Digitalkamera photographiert, nachdem von dieser Umgebung zuvor eine Referenzaufnahme ohne Kuh gefertigt wurde. Wenn es sich beim Gegenstand der Referenzaufnahme etwa um die Gitterbox eines Melkstandes handelt, an der zwischen den Melkterminen keine Änderungen vorgenommen werden, dann braucht solche Referenzaufnahme nur gelegentlich, jedenfalls nicht vor jeder Aufnahme der Kuh im Melkstand zu erfolgen.

Die bildliche Darstellung der Kuh erfolgt vor einem Hintergrund, dessen Einfärbung jedenfalls über dem Rückenbereich der Kuh zu den in ihrem Fell auftretenden Farbnuancen stark kontrastiert, wie im Falle eines grellgrünen Hintergrundes. Damit werden zugleich alle Hintergrund-Bildinhalte abgedeckt, da die für die Bildauswertung nicht benötigt werden. Ein entsprechendes Plakatieren der Standortumgebung der Kuh könnte diese allerdings davor abschrecken, den vorgesehenen Aufnahmeort zu betreten. Deshalb erfolgt die Hintergrundeinfärbung erst im Wege der Bildbearbeitung.

Die Referenzaufnahme und die aktuelle Aufnahme werden gegeneinander abgeglichen, nämlich pixelweise voneinander subtrahiert. Dazu dienen wiederkehrende Bildinhalte wie bauliche Einrichtungen als Orientierungsmarken. Das ergibt ein binäres Differenz-Bild, in welchem beispielsweise alle zwischen beiden Bildern übereinstimmenden Pixel, also insbesondere der nicht von der Kuh abgedeckte, in beiden Vergleichsaufnahmen kontrastfarbig übertünchte Hintergrund, aber auch stationäre Umgebungsgegebenheiten wie eine unbewegte Gitterbox, den binären Wert NULL erhalten. Für alle paarweise voneinander abweichenden Pixel der beiden Vergleichsaufnahmen verbleibt dann der Wert EINS. Das ergibt folglich eine Schwarz-Weiß-Darstellung der Kontur einer hellen Kuh vor dunklem Hintergrund, wenn etwa die NULL-Pixel schwarz und die EINS-Pixel weiß dargestellt werden.

Im Interesse einer Beschleunigung eines nun folgenden Scanvorganges ist ein, nach oben durch die Rückenlinie begrenzter, Streifen unter der Rückenlinie im Wege der automatischen Bildbearbeitung kontrastfarbig eingefärbt. Der ergibt sich, wenn der untere, unter der Rückenlinie gelegene Bereich des Hintergrundes unter der kontrastfarbigen Einfärbung bei der Bildbearbeitung hellfarbig, insbesondere weiß belegt wird, was zugleich die Seitenansicht der Kuh aufhellt.

Dann wird im Differenzbild der Verlauf der Rückenlinie relativ zu einer Referenzlinie für eine Aufeinanderfolge von vorgegebenen Mess-Punkten längs der Rückenlinie der Kuh farbgescannt, in einer Tabelle in kartesischen Koordinaten abgespeichert und als Liniendiagramm im Wege des Erfassens der Steigung der Regressionsgeraden auf Krümmung analysiert.

Dieses Aufnehmen und Auswerten der Rückenlinie der einzelnen, individuellen Kühe erfolgt vorzugsweise bei jedem der mehrmaligen täglichen Melktermine, um statistische Schwankungen in den Messergebnissen auszumitteln und frühzeitig Hinweise auf etwa einsetzende Lähmungserscheinungen bei einer Kuh zu bekommen. Denn eine resultierende, grenzwertige Krümmung der jeweiligen, einer bestimmten Kuh zugeordneten Punktefolge wird automatisch als das gesuchte Selektionskriterium, etwa als "wahrscheinlich lahmend", unter selbsttätiger Zuordnung zur Identifikation dieser Kuh protokolliert.

Für möglichst aussagekräftige, reproduzierbare Aufnahmen wird also angestrebt, die Kühe nacheinander in etwa übereinstimmend wiederkehrender stabiler Körperstellung zu photographieren. Das erfolgt vorzugsweise bei Stillstand einer Kuh nach Eintreten in eine Einplatz-Gitterbox bei oder unmittelbar nach Freigabe eines Futtertroges in automatisch an die Größe der Kuh angepasster Einstellung. Bei der Gitterbox handelt es sich bevorzugt um einen Teil eines Melk-Vollautomaten, wie er etwa Gegenstand der DE 6 99 18 442 T2 ist. Der wird von einer Kuh, wenn es für sie Zeit zum Melken und zum Futterfassen ist, selbständig aufgesucht, womit die Kuh die photographische Aufnahme mit gleich anschließender automatischer Bildverarbeitung samt binärer Auswertung auslöst.

Die erfindungsgemäße Einrichtung zum Selektieren einer lahmenden Milchkuh weist somit im Wesentlichen eine, bei einer Einzelstand-Gitterbox zu montierende, Digitalkamera auf, mit einer Fernauslösung für eine Aufnahme der Kuh bei Stillstand in einer Gitterbox - und für eine gelegentliche Referenzaufnahme der bloßen Gitterbox; mit einer rechnergesteuerten Bildbearbeitung zu Einfärben des Hintergrundes, pixelweiser Subtraktion der beiden Aufnahmen voneinander, Scannen der Rückenlinie der Kuh und zu klassierter Dokumentation der Krümmung dieser Rückenlinie.

Zusätzliche Alternativen und Weiterbildungen der erfindungsgemäßen Lösung ergeben sich aus den weiteren Ansprüchen und, auch unter Berücksichtigung von deren Vorteilen, aus nachstehender Beschreibung eines bevorzugten Realisierungsbeispieles zur Erfindung. In der Zeichnung zeigt:

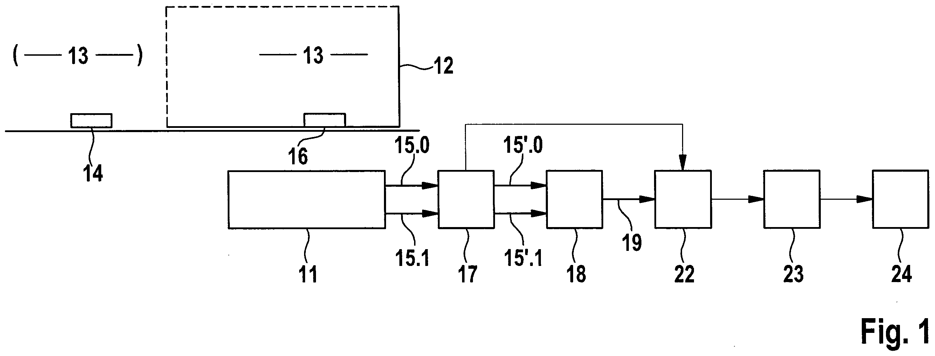

- Fig.1: nach Art eines Blockschaltbildes auf das Funktionswesentliche vereinfacht den stark abstrahiert skizzierten erfindungsgemäßen Ablauf zur vollautomatischen Anzeige einer als wahrscheinlich lahmend zu selektierenden Milchkuh aus einem Bestand,
- Fig.2: vor nachträglich eingefärbtem Hintergrund die Aufnahme einer in der Gitterbox eines Melk- und Fütterautomaten stehenden Kuh und
- Fig.3: das Differenzbild aus der Darstellung nach Fig.2 und einer entsprechenden Referenzaufnahme vor Eintreten der Kuh in die Gitterbox (nicht dargestellt).

Eine Kamera 11 ist bei oder an einer Gitterbox 12 montiert, die für eine einzelne, vorübergehend in möglichst definierter Stellung stillstehende Kuh 13 ausgelegt ist, etwa eingerichtet als vollautomatischer Melkstand mit individuell tiergesteuerter Einstellung und Freigabe einer Futterkrippe. Über einen Fernauslöser 14 kann gelegentlich, etwa von einer sich annähernden Kuh 13, manuell oder auch aus der rechner-programmgesteuerten Bildverarbeitung 17 heraus eine aktualisierte Referenzaufnahme 15.0 der freien Gitterbox 12 initiiert werden. Jedenfalls erfolgt über einen Zielauslöser 16 eine Aufnahme 15.1 mit der Kuh 13 in der Gitterbox 12, nämlich wenn die Kuh 13 ihre individuelle Fress- und Melk-Ruhestellung in der Gitterbox 12 eingenommen hat.

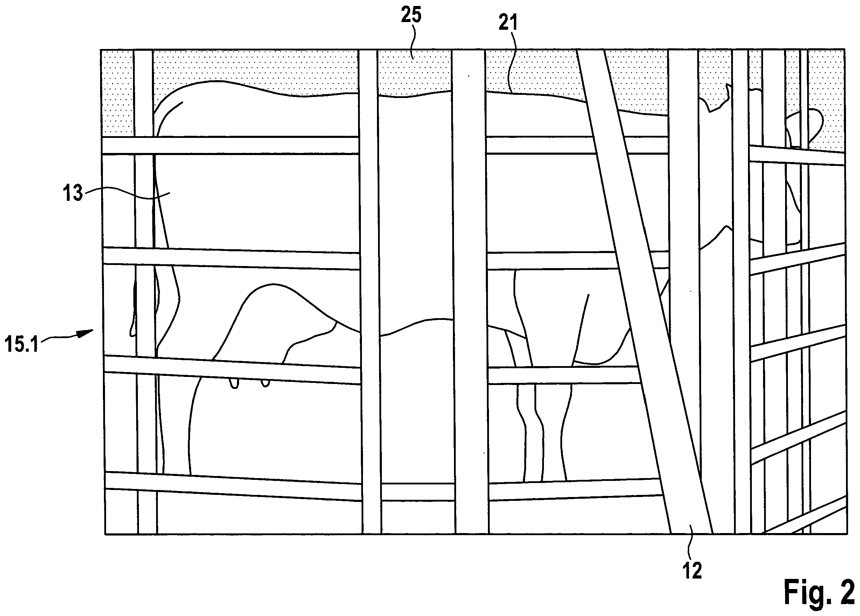

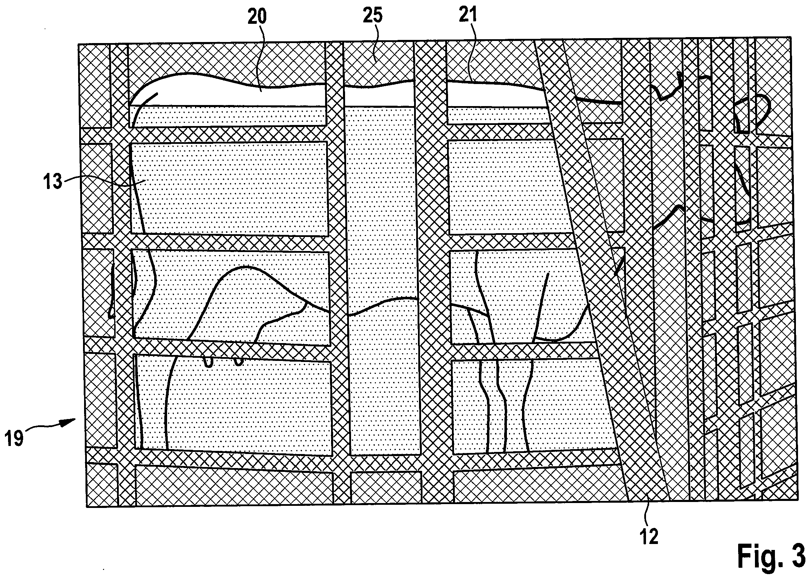

In einer von der Digital-Kamera 11 gespeisten, programmgesteuerten Bildbearbeitung 17 wird, jedenfalls hinter dem oberen Bereich der Kuh 13, in beiden zu vergleichenden Aufnahmen 15.1, 15.0 übereinstimmend der Hintergrund 25 (Fig.2) kontrastierend zu den typischerweise im Fell einer Kuh 13 auftretenden Farbschattierungen eingefärbt; womit zugleich die Bildinhalte des Hintergrundes übertüncht werden, um sie vom Bildvergleich auszunehmen. Dann wird durch einen pixelweise arbeitenden Differenzbildner 18 unter Verwendung von Orientierungspunkten aus der stationären Gitterbox 12 ein farbbinäres Bild 19 (Fig.3) von der Kontur wenigstens der Rückenpartie der Kuh 13 in der Gitterbox 12 erstellt. Die hier nicht interessierenden Hals- und Kopfbereiche der Kuh 13 können ausgeblendet werden. Zwischen den beiden Vergleichsaufnahmen 15'.0/15'.1 unverändert auftretende Bildbereiche (also insbesondere der eingefärbte Hintergrund 25 oberhalb der Rückenlinie 21 der Kuh 13; aber auch die von den Aufnahmen 15 erfassten, als Orientierungs- oder Positionsmarken nützlichen Stäbe der Gitterbox 12) werden in diesem Bild 19 dunkel dargeboten, Unterschiede in den bearbeiteten Vergleichsaufnahmen 15'.0/15'.1 dagegen hell. Die Seitenansicht der Kuh 13 im Differenzbild 19 erscheint aufgehellt, wenn unterhalb der bevorzugt grünen Einfärbung eine weiße Fläche erzeugt wird. Daraus resultiert dann auch ein zum Hintergrund 25 farblich kontrastierender Streifen 20 längs der Rückenlinie 21 der Kuh 13.

Solch ein nach oben durch die Rückenlinie 21 begrenzter Farbstreifen 20 erleichtert es, beim Abtasten mittels eines Scanners 22 vom dunklen Hintergrund her den Verlauf des oberen Randes des Streifens 20 und somit den Verlauf der Rückenlinie 21 an vorgegebenen Punkten hinter dem Hals der Kuh 13, oder gegebenenfalls auch bezüglich bestimmter Referenzpunkte an der Gitterbox 12, koordinatenmäßig zu erfassen. Die Krümmung einer durch diese Koordinaten verlaufenden Linie wird in einem Analysator 23, wie er etwa in einer mit den Abtast-Koordinaten gespeisten Tabellenkalkulation verfügbar ist, erfasst und bei Überschreiten eines voreingestellten Grenzwertes, zusammen mit der Individualisierung der Kuh 13 und einem Zeitstempel, in einem Speicher 24 unmittelbar, als Mittelwert aus mehreren Aufnahmen 15.1 und / oder als Veränderungs-Trend dokumentiert sowie auf einer Anzeige dargeboten.

Damit handelt es sich um eine für die Kuh stressfreie, frühestmögliche Erkennung einer zu behandelnden Klauenerkrankung, die andernfalls infolge gezielter Entlastung gerade der erkrankten Klauen zu einer Lahmheit führt, welche das gewöhnliche Verhalten des Tieres stark einschränkt; was zu schmerzbedingt verringerter Wasser- und Futteraufnahme und so zu sinkender Milchleistung führt.

Die Einrichtung zur Anzeige einer wahrscheinlich als lahmend aus einem Milchkuh-Bestand zur Behandlung zu selektierenden Kuh 13 weist also erfindungsgemäß in definierter räumlicher Relation zu einem von der Kuh 13 mehrmals täglich aufgesuchten automatischen Melkstand oder dergleichen Gitterbox 12 eine von der individuellen Kuh 13 bei Stillstand in der Gitterbox 12 mittels eines Zielauslösers 16 bei der Gitterbox 12 auslösbare Kamera 11 auf. Der ist ein Differenzbildner 18 für diese aktuelle digitale Aufnahme 15.1 und eine digitale Referenzaufnahme 15.0 nachgeschaltet, die bei gleicher Kameraeinstellung gelegentlich von der leeren Gitterbox 12 gemacht wurde. Ein auf automatischer Kurvenauswertung basierender Krümmungs-Analysator 23 liefert den Verlauf der im resultierenden Bild 19 gescannten Rückenlinie 21 der Kuh 13 unter Angabe eines Krümmungswertes oder einer Krümmungstendenz der Rückenlinie 21 einer individuellen Kuh 13 als deren Erkrankungsanzeige. Wenigstens eine Bildbearbeitung 17 dient einem optionalen Einfärben des Hintergrundes 25 der Aufnahmen 15.0, 15.1 und / oder eines durch die Rückenlinie 21 begrenzten, farblich kontrastierenden Streifens 20 im Differenz-Bild 19.

Das mittels solcher Einrichtung ausübbare Verfahren zur Anzeige einer wahrscheinlich als lahmend aus einem Milchkuh-Bestand zu selektierenden Kuh 13 beruht erfindungsgemäß im Wesentlichen darauf, möglichst mehrmals täglich mittels programmgesteuerter Bildverarbeitung 17 ein Differenzbild 19 aus einer digitalen Aufnahme 15.1 der in einer aktuellen, vorgegebenen Umgebung stehenden Kuh 13 und einer gelegentlichen digitalen Referenz-Aufnahme 15.0 dieser Umgebung ohne die Kuh 13 zu bilden und in diesem resultierenden Bild 19 automatisch die Krümmung des aktuellen und / oder des tendenziellen Verlaufes der Rückenlinie 21 der Kuh 13 zu analysieren, abzuspeichern und anzuzeigen; wobei zweckmäßigerweise die Aufnahmen 15.0, 15.1 vor einem gleichfarbigen Hintergrund 25 dargestellt werden, der mittels der handelsüblichen Bildbearbeitung 17 in den Aufnahmen 15.0, 15.1 eingefärbt wird; mittels derer auch in das resultierenden Differenz-Bild 19 längs der koordinatenmäßig abzutastenden Rückenlinie 21 ein von dieser begrenzter farbiger Streifen 20 eingebracht werden kann.

### Bezugszeichenliste

- 11: Kamera (zum Aufnehmen von 12; und von 13 in 12)
- 12: Gitterbox (für 13)
- 13: Kuh
- 14: Fernauslöser (für 15.0)
- 15: Aufnahmen (15.0 / 15.1 ohne / mit Kuh 13; 15 / 15' vor / nach 17)
- 16: Zielauslöser (für 11 durch 13 in 12; für 15.1)
- 17: Bildbearbeitung (für 15 zu 15'; und gegebenenfalls für 20 in 19)
- 18: Differenzbildner (für 15.0 - 15.1; gegebenenfalls für 15'0 - 15'1)
- 19: (Differenz-)Bild (hinter 18; mit 21)
- 20: Streifen (längs 21 in 19)
- 21: Rückenlinie (von 13)
- 22: Scanner (für 21 in 19)
- 23: Analysator (für 21 hinter 22)
- 24: Speicher (hinter 23)
- 25: Hintergrund (von 15')

## Patentansprüche

1. Verfahren zum Anzeigen einer als wahrscheinlich lahmend aus einem Milchkuh-Bestand zu selektierenden Kuh (13) durch Differenzbildung einer Aufnahme (15.1) einer Seitenansicht der Kuh (13) und einer Referenz-Aufnahme (15.0) ohne die Kuh (13), woraufhin in dem resultierenden Differenz-Bild (19) die Krümmung des Verlaufes der Rückenlinie (21) programmgesteuert analysiert, abgespeichert und angezeigt wird, wobei die Referenz-Aufnahme (15.0) nur gelegentlich von einer Gitterbox (12) eines automatischen Futter- und / oder Melkstandes genommen wird, wobei aber die jeweilige, individualisierte Kuh (13) wiederholt in dieser Gitterbox (12), infolge Anpassung des Futtertroges an die Größe der Kuh (13) in annähernd definiert wiederkehrender Körperstellung still stehend, aufgenommen wird, und wobei die zu vergleichenden Aufnahmen (15.0, 15.1) erst im Zuge der Bildbearbeitung jedenfalls im Rückenbereich der Kuh (13) einen gleichfarbigen Hintergrund (25) erhalten, der zu in ihrem Fell auftretenden Farbnuancen stark kontrastiert, mit der Gitterbox (12) als Referenzmarken für pixelweises Subtrahieren der Aufnahmen (15.0, 15.1) voneinander, woraufhin der daraus resultierende Verlauf der Rückenlinie (21) farbgescannt und als Liniendiagramm auf Krümmung analysiert wird.

2. Verfahren nach dem vorangehenden Anspruch, **dadurch gekennzeichnet, dass** die Aufnahmen (15.0, 15.1) einen hellen unter einem kontrastfarbigen Hintergrund (25) erhalten.

3. Verfahren nach dem vorangehenden Anspruch, **dadurch gekennzeichnet, dass** in das Differenz-Bild (19) längs der Rückenlinie (21) ein von dieser begrenzter kontrastfarbiger Streifen (20) eingebracht wird.

4. Verfahren nach einem der vorangehenden Ansprüche, **dadurch gekennzeichnet, dass** aus mehreren Ergebnissen von Analysen der Krümmung des Verlaufes der Rückenlinie (21) einer individuellen Kuh (13) ein Mittelwert abgespeichert und angezeigt wird.

5. Verfahren nach einem der vorangehenden Ansprüche, **dadurch gekennzeichnet, dass** aus mehreren Ergebnissen von Analysen der Krümmung des Verlaufes der Rückenlinie (21) einer individuellen Kuh (13) ein Veränderungstrend abgespeichert und angezeigt wird.

6. Einrichtung zum Ausüben wenigstens eines der Verfahren nach den vorangehenden Ansprüchen mit einer mittels eines Zielauslösers (16) auslösbaren Kamera (11), welcher ein Differenzbildner (18) für die aktuelle Aufnahme (15.1), und für eine gelegentliche Referenzaufnahme (15.0) ohne Kuh (13), nachgeschaltet ist, sowie ein Krümmungs-Analysator (23) mit Speicher (23) für Ermitteln und Anzeigen des aktuellen, eines über mehrere Aufnahmen gemittelten und / oder eines tendenziellen Verlaufes der Krümmung der im resultierenden Differenz-Bild (19) gescannten Rückenlinie (21) der Kuh (13), wobei die Kamera (11) in definierter räumlicher Relation zur Einplatz-Gitterbox (12) eines automatischen Melk- und / oder Futterstandes mit Freigabe eines Futtertroges nach automatisch der Größe der Kuh (13) sich anpassender Einstellung installiert ist, der wenigstens eine programmgesteuerte Bildbearbeitung (17) zum Einfärben des Hintergrundes (25) der Aufnahmen (15.0, 15.1) nachgeschaltet ist.

7. Einrichtung nach dem vorangehenden Anspruch, **dadurch gekennzeichnet, dass** der Kamera (11) wenigstens eine programmgesteuerte Bildbearbeitung (17) zum Einfärben eines durch die Rückenlinie (21) begrenzten Streifens (20) im Differenz-Bild (19) nachgeschaltet ist.

## Claims

1. Method for displaying a cow (13) to be selected from a group of dairy cows as suspected of going lame, by forming the difference between a picture (15.1) of a side view of the cow (13) and a reference picture (15.0) without the cow (13), whereupon in the resultant image of the difference (19) the curvature of the shape of the back line (21) is analysed in a program-controlled manner, stored and displayed, wherein the reference picture (15.0) is only occasionally taken of a barred box (12) of an automatic feeding stand and/or milking parlour, wherein a picture of the cow that is respectively singled out (13) is however repeatedly taken in this barred box (12), for reason of adapting the feeding trough to the size of the cow (13) standing still in an approximately defined recurring body position, and wherein the pictures (15.0, 15.1) to be compared are first given a uniformly coloured background (25) in the course of the image processing, at least in the back region of the cow (13), in stark contrast to nuances of colour occurring in the hide of the cow, with the barred box (12) providing reference markers for pixel-based subtraction of the pictures (15.0, 15.1) from one another, whereupon the shape of the back line (21) resulting from this is colour-scanned and analysed for curvature as a line diagram.

2. Method according to the preceding claim, **characterized in that** the pictures (15.0, 15.1) are given a light background (25) under a contrasting colour.

3. Method according to the preceding claim, **characterized in that** a strip (20) of a contrasting colour delimited from the back line (21) is introduced along the latter into the image of the difference (19).

4. Method according to one of the preceding claims, **characterized in that** a mean value from multiple results of analyses of the curvature of the shape of the back line (21) of an individual cow (13) is stored and displayed.

5. Method according to one of the preceding claims, **characterized in that** a trend of a change from multiple results of analyses of the curvature of the shape of the back line (21) of an individual cow (13) is stored and displayed.

6. Device for performing at least one of the methods according to the preceding claims, comprising a camera (11), which is triggered by means of an aim trigger (16) and is followed by a difference former (18) for the current picture (15.1), and for an occasional reference picture (15.0) without a cow (13), and also a curvature analyser (23) with a memory (23) for determining and displaying the current shape, a shape averaged over multiple pictures and/or a tendential shape of the curvature of the back line (21) of the cow (13) scanned in the resultant image of the difference (19), wherein the camera (11) is installed in a defined spatial relationship with a single-stall barred box (12) of an automatic milking parlour and/or feeding stand, with a feeding trough being enabled after a setting in which it adapts itself automatically to the size of the cow (13), and is followed at least by a program-controlled image processing (17) for colouring the background (25) of the pictures (15.0, 15.1).

7. Device according to the preceding claim, **characterized in that** the camera (11) is followed at least by a program-controlled image processing (17) for colouring a strip (20) delimited by the back line (21) in the image of the difference (19).

## Revendications

1. Procédé pour indiquer une vache (13) à sélectionner comme étant vraisemblablement boiteuse dans un troupeau de vaches laitières par détermination d'une différence entre une prise de vue (15.1) latérale de la vache (13) et une prise de vue de référence (15.0) sans la vache (13), la courbure de l'évolution de la ligne du dos (21) étant ensuite analysée, mémorisée et affichée de manière commandée par programme dans l'image de différence résultante (19),
dans lequel la prise de vue de référence (15.0) n'est prise qu'occasionnellement depuis un box grillagé (12) d'une salle de distribution de fourrage et/ou de traite automatique, dans lequel, la vache respective (13) individualisée est cependant reçue de manière répétée dans ledit box grillagé (12), suite à l'adaptation de la mangeoire à la taille de la vache (13), dans une position corporelle approximativement définie, récurrente et stationnaire, et dans lequel les prises de vue (15.0, 15.1) à comparer ne reçoivent un arrière-plan (25) d'une même couleur dans la zone du dos de la vache (13) qu'au cours du traitement d'image, lequel arrière-plan contraste fortement par les nuances de couleur apparaissant dans son pelage, le box grillagé (12) servant de repères pour soustraire les prises de vues (15.0, 15.1) les unes aux autres pixel par pixel, l'évolution qui en résulte de la ligne du dos (21) étant soumise à un balayage des couleurs et analysée du point de vue de sa courbure sous la forme d'un diagramme de lignes.

2. Procédé selon la revendication précédente, **caractérisé en ce que** les prises de vues (15.0, 15.1) sont dotées d'un arrière-plan clair sous un arrière-plan (25) présentant une couleur de contraste.

3. Procédé selon la revendication précédente, **caractérisé en ce qu'**une bande présentant une couleur de contraste (20) est introduite dans l'image de différence (19) le long de la ligne du dos (21), laquelle bande est délimitée par cette dernière.

4. Procédé selon l'une des revendications précédentes, **caractérisé en ce qu'**une valeur moyenne obtenue à partir de plusieurs résultats d'analyses de la courbure de l'évolution de la ligne du dos (21) d'une vache individuelle (13) est stockée et affichée.

5. Procédé selon l'une des revendications précédentes, **caractérisé en ce qu'**une tendance des variations obtenue à partir de plusieurs résultats d'analyses de la courbure de l'évolution de la ligne du dos (21) d'une vache individuelle (13) est stockée et affichée.

6. Dispositif pour la mise en oeuvre d'au moins l'un des procédés selon les revendications précédentes, comportant une caméra (11) qui peut être déclenchée au moyen d'un déclencheur cible (16) et qui est reliée en aval d'un dispositif de calcul de différence (18) pour la prise de vue actuelle (15.1) et pour une prise de vue de référence (15.0) occasionnelle obtenue sans vache (13), ainsi qu'un analyseur de courbure (23) muni d'une mémoire (23) pour déterminer et afficher la courbure actuelle d'une évolution moyennée sur plusieurs prises de vue et/ou d'une évolution tendancielle de la courbure de la ligne du dos (21) de la vache (13) balayée dans l'image de différence (19) résultante,
dans lequel la caméra (11) est installée selon une relation spatiale définie par rapport au box grillagé (12) d'une salle de traite et/ou de distribution de fourrage automatique avec déclenchement d'une mangeoire conformément à un réglage adapté automatiquement à la taille de la vache (13), en aval de laquelle est relié au moins un moyen de traitement d'image (17) commandé par programme pour colorer l'arrière-plan (25) des prises de vues (15.0, 15.1).

7. Dispositif selon la revendication précédente, **caractérisé en ce qu'**au moins un moyen de traitement d'image commandé par programme (17) est relié en aval de la caméra (11) pour colorer une bande (20) délimitée par la ligne du dos (21) dans l'image de différence (19).
